# EUROPEAN PATENT APPLICATION

(11) **EP 0 875 564 A1**
(43) Date of publication of application: **04.11.1998**
(21) Application number: 97201323.9
(22) Date of filing: 02.05.1997
(51) Int. Cl.: C12N 15/12, C12N 15/15, C07K 14/815, C07K 14/435, C07K 4/12, A61K 38/08, A61K 38/17, A61K 38/58, C07K 16/18, C07K 16/38, C07K 16/42

(54) **Biologically active extract from the leech 'Limnatis nilotica'**

(71) Applicant: Voerman, Gerard, 2930 Brasschaat (BE)
(72) Inventor: Voerman, Gerard, 2930 Brasschaat (BE)
(74) Representative: Smulders, Theodorus A.H.J., Ir.

(57) **Abstract**

Various anticoagulant and fibrinolytic substances derived from arhynchobdellous haematophagous leeches belonging to the family hirudinidae having caracteristics as described, or genetically engineered forms thereof, or an isoform, mutant, derivative, bioprecursor, or a pharmaceutically acceptable salt, of the natural or engineered molecule. The substances having low MW of between 0.1 and 10.0 kDa have biologic and therapeutic activities and are useful as a drug medicament.

## Description

### Background of the invention

Various substances extracted from leeches are known to have biological activity. These were reviewed (Sawyer, 1990) and essentially two groups of activity can be recognised: antithrombotic and fibrinolytic activities on the one hand, and enzymes and inhibitors on the other hand.

In the first group one finds: **Hirudin**, a thrombin inhibitor (Markwardt, 1956; 1988; Petersen, et al, 1984); **Hementin**, a fibrinolytic agent (Budzynski, et al, 1981; Kirschbaum & Budzynski, 1990); **Antistasin**, an inhibitor of coagulation factor Xa (Gasic, et al, 1983) which was reported to have antimetastatic properties as well; **Ghilanten**, a factor X inhibitor (Condra, et al, 1989; Blankenship, et al, 1990).

In the second group one finds: **Bdellin**, an inhibitor of trypsin and plasmin (Fritz & Krejci, 1976); **Eglin**, an inhibitor of chymotrypsin, elastase and Cathepsin G (Seemüller, 1979); **Orgelase**, an hyaluronidase (Sawyer, 1986).

More recently, additions in this field have been published in the patent literature: a fibrinolytic enzyme isolated from *Hirudo medicinalis* which splits ε-(γ-Glutamyl)-Lysin sequences (EP 0502876); a platelet adhesion inhibitor isolated from *Hirudo medicinalis* which inhibits collagen-induced platelet aggregation (EP 0 552 269); a thrombin inhibitor from the leech *Hirudinaria manillensis* (international patent application PCT/GB89/01345); an inhibitor of platelet aggregation from leeches of the *Hirudinidae* family (EP 0 348 208); an anticoagulant/modulator factor isolated from *Hirudo medicinalis* (EP 0352903); and a chymotrypsin- and elastase inhibitor from *Hirudinaria manillensis* (international patent application PCT/NL90/00046).

### Summary of the invention

During applicants' standard research work with monocultures of various bacterial species derived from leeches, it was surprisingly found that substances derived from the leech *Limnatis nilotica* showed that, next to new anti-coagulant and new fibrinolytic activity, a coagulation-**promoting** activity was present.

Further searching into this activity seemed to show that it arises from a body-derived substance of the leech under investigation, or derives from the association with one or more of the bacterial species associated with the leech. Applicant subsequently studied and characterized the various substances which have anti-coagulant, pro-coagulant and fibrinolytic activity, and this group of substances was named "Zaghadin".

The present description reveals the characterisation and specific activities of the substances.

Applicant specifically intends that these substances "Zaghadin", as invented and described herein, include such substances however derived, whether through sequential and block synthesis or through gene cloning and expression.

The subject invention provides various anticoagulant and fibrinolytic substances derived from arhynchobdellous haematophagous leeches belonging to the family hirudinidae having caracteristics as described herein, or genetically engineered forms thereof, or isoforms, mutants, derivatives, bioprecursors, or pharmaceutically acceptable salts, of the natural or engineered molecule. The substances having low MW of between 0.1 and 10.0 kDa have biologic and therapeutic activities and are useful as a drug medicament.

### Detailed description of the invention

The present invention concerns compositions of matter useful as anti-coagulants, coagulation-promoters and fibrinolytic substances for treatment of various human and/or veterinarial clinical disorders, related with **homeostasis**, such as, but not limited to **thrombotic, fibrinolytic, inflammatory** and **immunological** disorders. The composition of matter is derived from all body parts and secretions of the leech, inclusive saliva and gut-, intestinal- and skin secretions and mucus, or derived from bacterial species associated with the leech.

For the definition of isoforms or mutants, one has to understand that by biologic evolution enzymatic and other system-active molecules are subject to continuous phylogenetic development. It is in this understanding that we define isoform or mutant forms of this molecule.

### Description of isolation

Frozen *Limnatis nilotica* (300 g) were dehydrated in 94% ethylalcohol at room temperature. Three changes of total 400 ml. 100 ml of the ethanol extract was lyophilized in vials, after adding 300 ml destilled water. (One can also, as an alternative, use the chopped heads of these leeches, or use activated mucus secretions from the live leeches, by immersing them for one hour in 150 mM NaCl, 10 mM Arginin and 20 mM Phosphate buffer, at pH 7.0 at room temperature, or immersing the live leeches in 4.5% ethylalcohol for two hours, and collecting the large mucus secretions then produced, or from pure leech saliva.)

### Presence of anti- and procoagulant and fibrino(geno)lytic substances in leech extract

The extract contains two different substances having effect on the coagulation time, one substance with inhibition activity on chromogenic activity of thrombin (IIa) and one or more substances having effect on fibrino(geno)lysis.
A. Enhancing coagulation
B. Inhibiting coagulation
C. Inhibiting chromogenic thrombin activity
D. Enhancing fibrinolysis.

### Sub A. methods:

a). thrombin-time with purified fibrinogen (IIa +/- Ca²⁺)
b). thrombin-time with plasma fibrinogen (IIa +/- Ca²⁺)
c). Arvin-time with purified fibrinogen (IIa + Ca²⁺)

### Characterisation of the substance:

a). expired after dialysis (cut off at appr. 10 kD)
b). through 1 kD filter ( < 1 kD)

### Specificity:

coagulation-time induced by Arvin and thrombin both reduced. Possible explanation: Increase in polymerisation and/or crosslinking of fibrine. Effects on mass/length ratio are likewise improbable.

### Sub B methods:

a). thrombin-time with purified fibrinogen (IIa +/- Ca²⁺)
b). thrombin-time with plasma fibrinogen (IIa +/- Ca²⁺)
c). Arvin-time with purified fibrinogen (IIa + Ca²⁺)

### Characterisation of the substance:

present after dialysis (membrane with 10 kD cut-off)

### Specificity:

coagulation time induced by thrombin increased;
coagulation time as induced by Arvin no effect.

### Sub C methods:

a). Inhibition of chromogenic activity of thrombin.

### Characterisation of the substance:

b) Inhibition of thrombin activity is present in dialysed extract and in the filtrate after a 10 kD filter, but not in filtrates after 3 and 1 kD filters.

### Sub D Methods:

a) Fibrin clot lysis after addition of thrombin and Ca²⁺ in the presence and absence of t-PA, by measurement of time-wise reduction of A405nm. Solubility of the clot was measured at 405 nm and determined in mE. Measurements were made during for 5 hours at 32°C with 5 minutes intervals and were made with application of the Micro programme (Organon Technika).

### Methods

### Extracts:

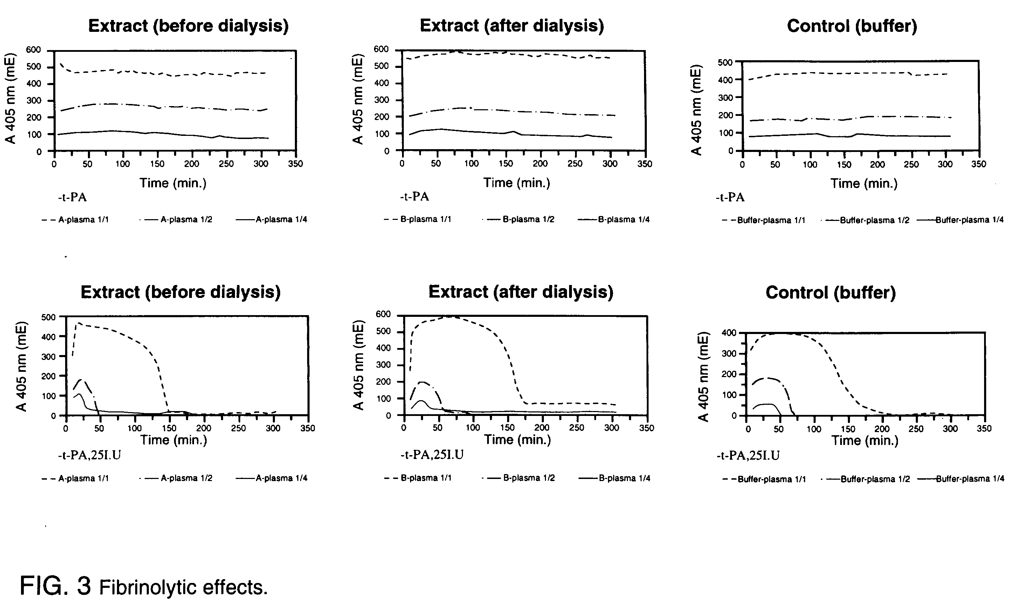

A. 4.5% ethylalcohol extract, centrifuged for 20 minutes at 2000 G was divided into portions and frozen at -20°C.
B. 4.5% ethylalcohol, dialysed against 50 mM triethanolamine (TEA), 100 mM NaCl at pH 7.5.
C. Various filtrates.
D. The extract before (A) as well as after dialysis (B) was mixed with plasma dilutions (1 : 1) (see Fig 3). Dialysis- and dilution buffer: 50 mM triaethanolamine (TEA), 100 mM NaCl, pH 7.5.

### Thrombin-time in plasma/extract

Determination on automatic coagulation machine ACL (Instrumentation Laboratory). 50 µl purified bovine thrombin, Dade, 100 NIHE/ml, was added to 100 µl of the mixture of citrate plasma and the extract (1 : 1), and stored at -20°C and diluted for use to 5 NIHE/ml. Measurement of coagulation time at 31°C. End concentration of extract in the determination was 33.33%.

### Thrombin-time in purified fibrinogen/extract

Determination on ACL (as above). 50 µl purified bovine thrombin, Dade, 100 NIHE/ml, was added to a mixture of 100 µl fibrinogen/extract mixture (1 : 1) (Fibrinogen Sigma T-6759, 13.2 µM in 50 mM TEA, 100 mM NaCl at pH 7.5) and extract. Measurement of coagulation time at 37°C. End concentration of extract in the determination was 33.33%.

Thrombin was diluted, either in a) 50 mM TEA, 100 mM NaCl, pH 7.5, or in b) 50 mM TEA, 100 mM NaCl, 40 mM CaCl₂, pH 7.5.

### Arvin-time in purified fibrinigen/extract

As described for thrombin-time in purified fibrinogen /extract (see above). Arvin snake-poison (Knoll) 1500 NIHE per ml, diluted to 10 NIHE/ml with TEA buffer + CaCl₂.

### Chromogenic determination of thrombin inhibition

Determination in microtiterwells. Dilutions of thrombin (human thrombin, Kordia HT 690 L, -1 µM in 50 mM TEA, 180 mM NaCl, 0.1% Ovalbumine pH 7.5) were mixed with the extract (1 : 1). From this mixture, a portion of 20 µl was added to a microtiterwell with 165 µl of the ovalbumine buffer as mentioned. Reaction was started by adding 25 µl Pefachrome TH, 2.2 mM, Pentafarm. Determination of p-nitroaniline was measured at 405 nm during 20 times 60 seconds at 20°C by application of the Micro programme (Organon Technika). Calculation of data in A 405 mE/sec. End concentration of extract during measurement was 4.8%.

### Filtration and lyophilisation

Macrosep filters (Filtron) of 10, 3, and 1 kD were used for filtration of the extract before dialysis. Material was lyophilized after filtration and subsequently resolubilized in respectively 1/5, 1/3 and 1/2 of the original volume in 10 mM TEA, pH 7.5.

### Presence of fibrinolytic components in the extract

In microtiter wells:
10 µl humane thrombin-Kordia Ht 690 L-125 nM, with end contration of 10 nM;
10 µl CaCl₂, 312.5 mM, with end concentration of 25 nM; 105 µl extract-plasma +/- t-PA.

### Examples

**Table I**

| Enhanced/Inhibited effect on coagulation as determined with thrombin-time | | |
|---|---|---|
| | - Ca²⁺ | + Ca²⁺ |
| A-NP | 15.2 | 9.6 |
| B-NP | 25.2 | 17.2 |
| C-NP | 18.2 | 14.1 |
| A-Fbg | 20.9 | 16.5 |
| B-Fbg | 56.2 | 52.3 |
| C-Fbg | 40.3 | 20.7 |
| A= extract before dialysis; B= extract after dialysis against 50 mM TEA, 100 mM NaCl, pH 7.6; c= buffer (50 mM TEA, 100 mM NaCl, pH 7.5); NP= Pooled citrated plasma; Fbg= purified fibrinogen (see Methods); thrombin diluted in TEA buffer +/- Ca²⁺ (see Methods). | | |

**Table II**

| Enhanced/Inhibited effect on coagulation as determined with Arvin-time | |
|---|---|
| + Ca²⁺ | sec |
| A-Fbg | 10.8 |
| B-Fbg | 25.3 |
| C-Fbg | 23.7 |
| A= extract before dialysis; B=extract after dialysis against 50mM TEA, 100 mM NaCl, pH 7.5; C= buffer (50 mM TEA, 100 mM NaCl, pH 7.5); Fbg= purified fibrinigen (see Methods); Arvin diluted in TEA buffer + Ca²⁺ (see Methods). | |

**Table III**

| Coagulation enhancing effect as determind by thrombin-time in filtrate of extract after filtration by 1 kD filter. | |
|---|---|
| + Ca²⁺ | sec |
| Fil-NP | 10.6 |
| C-NP | 17.5 |
| Fil-Fbg | 13.6 |
| C-Fbg | 34.4 |
| Fil=filtrate (see Methods); C= buffer (50 mM TEA, 100 mM NaCl, pH 7.5); NP= pooled citrate plasma; Fbg= purified fibrinogen (see Methods); thrombin diluted in TEA buffer + Ca²⁺ (see Methods). | |

**Table IV**

| Coagulation enhancing effect as determined by Arvin-time in filtrate of extract after filtration by 1 kD filter. | |
|---|---|
| + Ca²⁺ | sec |
| Fil-Fbg | 11.0 |
| C-Fbg | 21.9 |
| Fil= filtrate (see Methods); C= buffer (50 mM TEA, 100 mM NaCl, pH 7.5); Fbg= purified fibrinogen (see Methods); Arvin diluted in TEA buffer + Ca²⁺ (see Methods). | |

### Conclusions

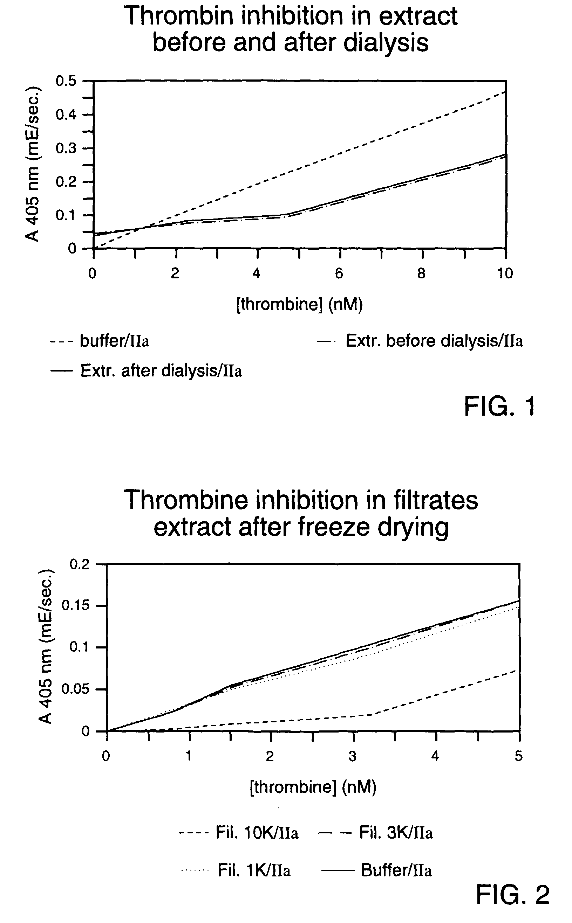

- From Table I derives the fact that the leech extract contains a component which increases the coagulation time, as well as a component which reduces the coagulation time. This was found for both plasma and purified fibrinogen in the presence and absence of Ca²⁺. The coagulation time reducing component is dialysable, whereas the coagulation time increasing component is not dialysable, at least partly.
- From Table II derives the fact that the coagulation time reducing component is not specific for thrombin: the Arvin-time is reduced in the extract before dialysis. The increase in coagulation time with Arvin-time was not found in the extract after dialysis; therefore, the coagulation time increasing component is specific for thrombin.
- From Table III derives the fact that the coagulation time reducing effect was filterable by the 1 kD filter. Therefore, this component has a MW < 1kD.
- Table IV confirms the fact that the thrombin-time reducing effect was also present in Arvin-time experiments; therefore, this is not specific for thombin.
- From Fig 1 derives the fact that the thrombin chromogenic activity is inhibited by the extract before as well as after dialysis.
- From Fig 2 derives the fact that the inhibitor of thrombin chromogenic activity does not pass the 1 and 3 kD filters, but passes (at least partly) the 10 kD filter.
- From Fig 3 derives the fact that a fibrino(geno)lytic effect is present before and after dialysis in plasma dilutions of 1/2 and 1/4 dilutions (-t-PA).

From these observations it can be concluded that the *L*. *nilotica* extract contains:
a). a **coagulation enhancing** component, with a MW of less than 1 kD,reducing both the Arvin- as well as the thrombin-time, probably as a consequence of an increase in polymerisation and/or crosslinking of fibrine.
b).a **coagulation inhibiting** component with a MW of over 3 kD, which reduces thrombin-time, but not Arvin-time. c). a **coagulation inhibiting** component inhibiting the enzymatic activity of thrombin. *(Components under b) and c) are presumably identical.)*
d) (a) **fibrinolytic components(s)** in the extract in plasma dilutions of 1/2 and 1/4 (-t-PA). This can be seen in Fig 3.

### Literature

Autrum H.: Hirudineen. Systematik. In Bronn's Klassen und Ordnungen des Tierreichs, Bd. 4 abt. III, Buch 4, T. 1 1936: 1-96.

Blaise M.: Accidents occasionnes par les sang-sues. J Med Vet militaire 1874-1875; 10.

Blankenship D.T., Brankamp R.G., Manley G.D., Cardin A.D.: Amino sequence of ghilanten: anticoagulant-antimetastatic principle of the South-American leech Haementeria ghilianii. Biophys Res Comm 1990; 166: 1384.

Budzynski A.Z., Olexa S.A., Brizuela B.S., Sawyer R.T., Stent G.S.: Anticoagulant and fibrinolytic properties of salivary proteins from the leech Haementeria ghilianii. Proc Soc Exp Biol Med 1981; 168: 266.

Condra C., Nutt E., Petrowski C.J., Simpson E., Friedman P.A., Jacobs J.W.: Isolation and structural characterisation of a potent inhibitor of coagulation factor Xa from the leech Haementeria ghilianii. Thromb Haemost 1989; 61: 437.

Edman P.: Acta Chem Scand 1956; 10: 761-768.

Fritz H., Krejci K.: Trypsin-plasmin inhibitors (Bdellins) from leeches. Meth Enzymol 1976; 45: 797.

Gasic G.J., Viner E.D., Budzynski A.Z., Gasic G.P.: Inhibition of lung tumour colonisation by leech salivary gland extracts from haementeria ghilianii. Cancer Research 1983; 43: 1633.

Ghedia S.: Contribution à l'étude des Hirudinees. Thesis, Sidi Thabet, Tunisie, 1984.

Hewick R.M., Hunkapiller M.W., Hood L.E., Dreyer W.J.: J Biol Chem 1981, 15: 7990-7997.

Harant H.: Essai sur les Hirunidées. Arch Soc Sci Montpellier 1927, 10 : 1-76.

Ilse D., Edman P.: Aust J Chem 1963, 16: 411-416.

Jarry D.: Sur la presence de Limnatis nilotica dans un oued meditarraneen: la baillaurie a Banyuls-sur-Mer. Bull Soc Zool Fr 1959, 84; 73-76.

Keegan H.L., Radke G., Murphy D.A.: Nasal leech infestation in man. Am J Trop Med Hyg 1970, 19-6: 1029-1030.

Kirschbaum N.E., Budzynski A.Z.: A unique proteolytic fragment of human fibrinogen containing the Aa-COOH-terminal domain of the native molecule. J Biol Chem 1990; 265: 489.

Markwardt F.: Die antagonistische Wirkung des Hirudins gegen Thrombin in vivo. Naturwissenschaften 1956; 43: 111.

Markwardt F.: Hirudin as an inhibitor of Thrombin. Meth Enzymol 1970; XIX: 924.

Markwardt F.: The come-back of hirudin: an old established anticoagulant agent. Folia Haematol 1988; 115: 10-23.

Mouquin-Tandon A.: Monographie de la famille des Hirunidées. Paris, 1846.

Neveu-Lemaire: Traité d'entomologie médicinale et véterinaire. 1938.

Petersen T.E., Roberts H.R., Sotrup-Jensen L., Magnusson S.: Primary structure of hirudin, a thrombin-specific inhibitor. In: Protides of the biological fluids. Ed: Peters. Pergamon Oxford 1975: 145-149.

Seemüller U.: Inhibitoren aus dem Blutegel Hirudo medicinalis mit Hemmwirkung gegen chymotrypsin, subtilisin, und die menschlichen Granulozytenproteasen elastase und kathepsin G. Ph D Dissertation, Munich University, 1979.

Turner F.M.: Pharyngial leeches. The Lancet 1969, dec '27, 1400-1401.

## Claims

1. A substance having an effect on blood coagulation, fibrinolysis or fibrinogenolysis, wherein said substance is derived from the leech *Limnatis nilotica* or from bacteria associated with the leech *Limnatis nilotica*.

2. A substance according to claim 1, having a MW of less than 1 kD and derived from the leech *Limnatis nilotica* or its associated bacteria, having a coagulation-time reducing activity.

3. A substance as under claim 2, which is not specific for thrombin.

4. A substance according to claim 1, having a MW of over 3 kD and derived from the leech *Limnatis nilotica* or its associated bacteria, having a coagulation-time increasing activity.

5. A substance as under claim 4, which is specific for thrombin.

6. A substance according to claim 1, derived from the leech *Limnatis nilotica* or its associated bacteria, having a inhibiting effect on the chromogenic activity of thrombin.

7. A substance as under claim 6, which has a MW of less than 10 kD.

8. A substance having the characteristics as those mentioned under claim 4, 5, 6 and 7 together.

9. A substance according to claim 1, derived from the leech *Limnatis nilotica* or its associated bacteria, which dissolves fibrin/fibrinogen.

10. A substance according to anyone of the foregoing claims for use as a therapeutic.

11. A pharmaceutical composition comprising a substance according to anyone of claims 1 to 9, together with suitable excipients.

12. Use of a substance according to anyone of the claims 1 to 9 in the preparation of a medicament for the treatment of bloodclotting disorders, disorders of immunological kind and of inflammatory diseases, or the use in cardio-pulmonary surgical therapies.

13. A nucleic acid molecule encoding at least a part of a proteinaceous or polypeptide-like substance according to substances from claims 1 to 9.

14. An expression vector comprising a nucleic acid molecule according to claim 13, together with a suitable control element for expression.

15. An expression system for expressing at least part of a substance according to claims 1 to 9, comprising an expression vector according to claim 14 and an expression mechanism.

16. An expression system according to claim 15, wherein the expression system is a recombinant host cell.

17. An antibody which specifically recognizes a substance according to anyone of claims 1 to 9.

18. An anti-idiotypic antibody mimicking at least an epitope of a substance according to anyone of claims 1 to 9.

19. An anti-idiotypic antibody mimicking at least an activity of a substance according to anyone of claims 1 to 9.
